Europäisches Patentamt

European Patent Office

Office européen des brevets

(19)

(11) Veröffentlichungsnummer: **0 023 355
B1**

(12)                 **EUROPÄISCHE PATENTSCHRIFT**

(45) Veröffentlichungstag der Patentschrift :
21.11.85

(51) Int. Cl.⁴ : **A 61 K   9/00, A 61 K 31/475**

(21) Anmeldenummer : 80104399.3

(22) Anmeldetag : 25.07.80

(54) Arzneimittellösungen und Verfahren zu deren Herstellung.

(30) Priorität : 26.07.79 DE 2930369

(43) Veröffentlichungstag der Anmeldung :
04.02.81 Patentblatt 81/05

(45) Bekanntmachung des Hinweises auf die Patenterteilung : 21.11.85 Patentblatt 85/47

(84) Benannte Vertragsstaaten :
AT BE CH FR GB IT LI NL

(56) Entgegenhaltungen :
DE-A- 2 134 671
DE-A- 2 735 587
US-A- 3 567 828
CHEMICAL ABSTRACTS, Band 84, Nr. 23, 23. Juni
1976, Seite 38, Nr. 159746d Columbus, Ohio, U.S.A. M.
WILIMOWSKI et al.: "Studies on the synergism of
some hypotensive drugs with ethanol"
EXPERIENTIA, Band 34, 15. Mai 1978, Seiten 637-639
Basel, CH. P.E. ZÜGER et al.: "Inhibition of reserpineinduced PGO waves in the cat by ergot derivatives"
JOURNAL OF CHROMATOGRAPHY, Band 170, Nr. 2,
11. März 1979, Seiten 399-404 Elsevier Scientific Publ.
Comp. Amsterdam, NL A. YOSHIDA et al.: "Etude par
chromatographie liquide à haute pression des alcaloïdes de l'ergot »

(73) Patentinhaber : Dr. Rentschler Arzneimittel GmbH &
Co.
Postfach 320 Mittelstrasse 18
D-7958 Laupheim (DE)

(72) Erfinder : Lahr, Wolfgang
Hopfenweg 18
D-7958 Laupheim (DE)

(74) Vertreter : Sandmair, Kurt, Dr. et al
Patentanwälte Schwabe, Sandmair, Marx Stuntzstrasse 16
D-8000 München 80 (DE)

**Beschreibung**

Die Erfindung betrifft stabile Lösungen von Rauwolfia-Alkaloiden und deren therapeutisch verträglichen Salzen allein oder zusammen mit anderen geeigneten Arzneiwirkstoffen in üblichen organischen Lösungsmitteln und/oder Wasser, sowie ein Verfahren zur Herstellung derartiger Lösungen.

In der modernen Chemotherapie nehmen Lösungen einen bedeutenden Platz ein, weil sie gegenüber den festen Darreichungsformen leichter einnehmbar und individuell dosierbar sind. Darüber hinaus weisen Arzneimittelzubereitungen mit gelöstem Wirkstoff gegenüber den festen Zubereitungen Vorteile, wie schnelleren Wirkungseintritt und höhere Resorptionsquote, auf.

In der Therapie des Blutdruck-Hochdruckes sind Rauwolfia-Alkaloide allein oder in Kombinationen mit anderen Antihypertonika von vorrangiger Bedeutung. Da Rauwolfia-Alkaloide aber in Lösungen zur Instabilität neigen und andere Antihypertonika nur schwer in Lösung zu bringen sind, werden im gebräuchlichen Arzneimittelsortiment nur feste Darreichungsformen angeboten, die diese Arzneistoffe enthalten (vgl. Rote Liste 1979, lfd. Nr. 16 038 bis 16 099).

Um Reserpin in Lösung zu bringen und die Lösung stabil zu halten, sind bereits verschiedene Vorschläge gemacht worden, die jedoch Nachteile mit sich bringen. So wurde zwar von Pötter und Voigt (Die Pharmazie, 22, (1967), S. 436) eine bei 37 °C über drei Jahre, bzw. eine bei Raumtemperatur über fünf Jahre stabile Reserpin-Injektionslösung beschrieben, die Thioharnstoff als Antioxydans und Methionin zur Ausschaltung von Schwermetallionen verwendet. In einem anderen Vorschlag von Asker et. al. (Die Pharmazie, 26, (1971), S. 90) wird p-Aminobenzoesäure als UV-Absorber empfohlen. Abgesehen davon, daß beide Vorschläge nur für Injektionslösungen bestimmt sind, und ihre Verwendbarkeit für orale Lösungen offen ist, eignen sie sich wegen der Verwendung des als kanzerogen eingestuften Thioharnstoffes (vgl. D. Schmähl, Dtsch. Ärzteblatt, 74, (1977), S. 89) bzw. der allergenen p-Aminobenzoesäure (H. J. Bandmann, W. Dohn, « Die Epicutan-Testung », Verlag J. F. Bergmann, München, 1967, S. 65) nicht für den Gebrauch in der Humantherapie.

Dieser Sachverhalt wird dadurch bestätigt, daß ein singuläres Reserpin-Tropfenpräparat (Serpasil®, vgl. Rote Liste 1979, lfd. Nr. 16 041) zur Maskierung auftretender Verfärbungen gelb angefärbt wird und dem ausgewiesenen Verfallsdatum zufolge nur begrenzt haltbar ist.

Für schwerlösliche Diuretika sind keine oral zu verabreichend Lösungen bekannt.

Aus der US-A-3 567 828 ist eine pharmazeutische Zubereitung zur Erniedrigung des Blutdrucks bekannt, die als aktive Bestandteile Reserpin oder ein wasserlösliches, pharmazeutisch verträgliches Salz davon, Dihydroergocristin oder ein wasserlösliches, pharmazeutisch verträgliches Salz davon und N-(cis-2,6-Dimethylpiperidino)-4-chlor-3-sulfamoylbenzamid enthalten. Es werden in dieser Patentschrift neben festen Darreichungsformen zwar auch injizierbare Lösungen der genannten Wirkstoffe erwähnt, jedoch wird lediglich ganz allgemein angegeben, daß zu ihrer Herstellung übliche Hilfsstoffe zugesetzt werden. Diese Injektionslösungen sind weder in ihrer Zusammensetzung offenbart, noch wird irgendeine Aussage über ihre Stabilität gemacht.

Der Erfindung lag daher die Aufgabe zugrunde, in üblichen organischen Lösungsmitteln und/oder Wasser schwerlösliche Rauwolfia-Alkaloide, sowie gegebenenfalls auch schwerlösliche Antihypertonika und/oder Diuretika in Lösung zu bringen und die erhaltene Lösung gleichzeitig zu stabilisieren, um stabile, oral verabreichbare oder injizierbare Lösungen zu erhalten.

Diese Aufgabe wurde nun dadurch gelöst, daß diese Lösungen als Lösungsvermittler und Stabilisator Methansulfonsäure oder Ethansulfonsäure in einer Menge von 0,05 bis 2,0 Gewichtsprozent sowie gegebenenfalls als andere Arzneiwirkstoffe schwerlösliche Antihypertonika und/oder Diuretika enthalten.

Die Erfindung ermöglicht, Rauwolfia-Alkaloide in Lösung zu bringen zu stabilisieren und gleichzeitig andere therapeutisch erwünschte, schwer lösliche Antihypertonika, insbesondere Mutterkornalkaloide, und Diuretika in Lösung zu bringen. Auf diese Weise können optimale Arzneiwirkstoffmischungen, wie sie vorzugsweise in der Hochdrucktherapie eingesetzt werden, auch in der erwünschten oralen oder injizierbaren löslichen Form angeboten werden. Besonders bevorzugt sind die in den Patentansprüchen 4, 7 und 8 beschriebenen stabilen Lösungen.

In den erfindungsgemäßen Lösungsmittelgemischen können schwer lösliche Diuretika und Antihypertonika zusammen mit Rauwolfia-Alkaloiden gelöst werden. Es wurde nämlich gefunden, daß durch Zugabe von Methan- oder Äthansulfonsäure in einer Menge von 0,05-2,0 Gewichtsprozent, vorzugsweise Methansulfonsäure, Rauwolfia-Alkaloide in diesen Lösungen über längere Zeit stabil bleiben. Dies läßt sich in üblichen Stabilitätsuntersuchungen nachweisen und ist darüber hinaus an der Nichtverfärbung der Rauwolfia-Alkaloide in den Lösungen ablesbar (vgl. Pötter, Voigt, Die Pharmazie, 22, 8 (1967). S. 436 ff., S. 437).

Dies ist um so überraschender, als Säuren in Lösungen gewöhnlich nur zur Verbesserung der Löslichkeit Verwendung finden, nicht aber zur Stabilität der Arzneiwirkstoffe in diesen Lösungen beitragen. So verwenden beispielsweise Pötter und Voigt (a. a. O.) Säuren wie Citronen-, Wein-, Ascorbin-, Essig-, Salz- und Phosphorsäure, um die Löslichkeit von Reserpin zu verbessern, benötigen aber gleichzeitig zur Stabilisierung des Reserpins in der Lösung weitere Zusätze wie Thioharnstoff und Methionin, die den oben beschriebenen Bedenken unterliegen (vgl. Schmähl, a. a. O.).

Die Herstellung der erfindungsgemäßen Lösungen erfolgt in der Weise, daß man Rauwolfia-Alkaloide und deren Salze allein oder zusammen mit anderen geeigneten Arzneiwirkstoffen in organischen Lösungsmitteln und/oder Wasser löst und 0,05 bis 2,0 Gewichtsprozent Methan- oder Ethansulfonsäure zusetzt.

Als Alkylsulfonsäure wird vorzugsweise Methansulfonsäure und als Lösungsmittel werden ein- oder mehrwertige Alkohole, wie Propandiol, Äthanol oder Glycerin in einer Menge von weniger als 1 bis 100 %, sowie entmineralisiertes Wasser verwendet. Der $p_H$-Bereich wird unter Verwendung einer Base, vorzugsweise Ammoniak, auf einen Wert von 2,5 bis 3,5 eingestellt.

Die gewünschten Arzneiwirkstoffe werden in geeigneter Form und dem therapeutischen Verwendungszweck angepaßter Menge eingebracht.

Die jeweiligen Mengen der gelösten Wirkstoffe und stabilisierenden Lösungsmittel richten sich nach der beabsichtigten therapeutischen Wirkung und ihrer Löslichkeit in den gewählten Lösungsmitteln bzw. deren Gemischen.

Beispiel

| | | |
|---|---|---|
| 0,1 | g | Reserpin |
| 20,0 | g | Propandiol |
| 0,2 | g | Methansulfonsäure |
| 0,13 | g | Ammoniak |
| ad 100,0 | g | Wasser |

**Patentansprüche**

1. Stabile Lösungen von Rauwolfia-Alkaloiden und deren therapeutisch verträglichen Salzen allein oder zusammen mit anderen geeigneten Arzneiwirkstoffen in üblichen organischen Lösungsmitteln und/oder Wasser, dadurch gekennzeichnet, daß sie als Lösungsvermittler und Stabilisator Methansulfonsäure oder Ethansulfonsäure in einer Menge von 0,05 bis 2,0 Gewichtsprozent sowie gegebenenfalls als andere Arzneiwirkstoffe schwerlösliche Antihypertonika und/oder Diuretika enthalten.

2. Stabile Lösungen nach Anspruch 1, dadurch gekennzeichnet, daß als Arzneiwirkstoff Reserpin enthalten ist.

3. Stabile Lösungen nach Anspruch 2, dadurch gekennzeichnet, daß als Arzneiwirkstoffe Reserpin und Diuretika enthalten sind.

4. Stabile Lösungen nach Anspruch 3, dadurch gekennzeichnet, daß als Arzneiwirkstoffe Reserpin und Clopamid [N-(cis-2,6-Dimethylpiperidino)-4-chlor-3-sulfamoylbenzamid] enthalten sind.

5. Stabile Lösungen nach Anspruch 2, dadurch gekennzeichnet, daß als Arzneiwirkstoffe Reserpin und andere übliche Antihypertonika enthalten sind.

6. Stabile Lösungen nach Anspruch 5, dadurch gekennzeichnet, daß als Arzneiwirkstoffe Reserpin und Mutterkornalkaloide enthalten sind.

7. Stabile Lösungen nach Anspruch 6, dadurch gekennzeichnet, daß als Arzneiwirkstoffe Reserpin und ein Gemisch von Dihydroergocristin, Dihydroergocornin, Dihydroergokryptin als Methansulfonate, vorzugsweise im Verhältnis 1 : 1 : 1 enthalten sind.

8. Stabile Lösungen nach Anspruch 2, dadurch gekennzeichnet, daß als Arzneiwirkstoffe Reserpin, Dihydroergocristin-Methansulfonat und Clopamid [N-(cis-2,6-Dimethylpiperidino)-4-chlor-3-sulfamoylbenzamid] enthalten sind.

9. Stabile Lösungen nach Anspruch 1 bis 8, dadurch gekennzeichnet, daß als Lösungsvermittler und stabilisierendes Mittel Methansulfonsäure enthalten ist.

10. Verfahren zur Herstellung von stabilen Lösungen nach Anspruch 1 bis 9, dadurch gekennzeichnet, daß man Rauwolfia-Alkaloide und deren Salze allein oder zusammen mit anderen geeigneten Arzneiwirkstoffen in organischen Lösungsmitteln und/oder Wasser löst und 0,05 bis 2,0 Gewichtsprozent Methan- oder Ethansulfonsäure, vorzugsweise Methansulfonsäure, zusetzt.

**Claims**

1. Stable solutions of rauwolfia alkaloids and their therapeutically acceptable salts alone or together with other suitable active pharmaceutical substances in conventional organic solvents and/or water, characterised in that they contain, as solubilising agents and stabilizers, methane sulphonic acid or ethane sulphonic acid in an amount of 0.05 to 2.0 percent by weight and optionally antihypertonics and/or diuretics as other active pharmaceutical substances.

2. Stable solutions as claimed in Claim 1, characterised in that reserpine as an active pharmaceutical substance.

3. Stable solutions as claimed in Claim 2, characterised in that they contain reserpine and diuretics as active pharmaceutical substances.

4. Stable solutions as claimed in Claim 3, characterised in that they contain reserpine and clopamide [N-(cis-2,6-dimethylpiperidino) 4-chloro-3-sulphamoylbenzamide] are contained as active pharmaceutical substances.

5. Stable solutions as claimed in Claim 2, characterised in that they contain reserpine and other convention antihypertonics as active pharmaceutical substances.

6. Stable solutions as claimed in Claim 5, characterised in that they contain reserpine and rye ergot alkaloids as active pharmaceutical substances.

7. Stable solutions as claimed in Claim 6, characterised in that they contain reserpine and a mixture of dihydroergocristin, dihydroergocornin, dihydroergokryptin as methane sulphonates, preferably in the ratio of 1 : 1 : 1 as active pharmaceutical substances.

8. Stable solutions as claimed in Claim 2, characterised in that they contain reserpine, dihydroergocristin-methane sulphonate and clopamide [N-(cis-2,6-dimethylpiperidino)-4-chloro-3-sulphamoylbenzamide] as active pharmaceutical substances.

9. Stable solutions as claimed in Claims 1 to 8, characterised in that methane sulphonic acid is contained as a solubilizing and stabilizing agent.

10. A method of producing stable solutions as claimed in Claims 1 to 9, characterised in that rauwolfia alkaloids and their salts are dissolved alone or together with other suitable active pharmaceutical substances in organic solvents and/or water and 0.05 to 2.0 percent by weight of methane or ethane sulphonic acid, preferably methane sulphonic acids, is added.

**Revendications**

1. Solutions stables d'alcaloïdes de Rauwolfia et de leurs sels thérapeutiquement compatibles, seuls ou conjointement avec d'autres substances actives médicamenteuses appropriées dans des solvants organiques habituels et/ou dans l'eau, caractérisées en ce que, comme unisseur et stabilisant, elles contiennent de l'acide méthane-sulfonique ou de l'acide éthane-sulfonique en une quantité de 0,05 à 2 % en poids, ainsi qu'éventuellement des diurétiques et/ou des antihypertoniques difficilement solubles comme autres substances actives médicamenteuses.

2. Solutions stables selon la revendication 1, caractérisée en ce que, comme substance active médicamenteuse, elles contiennent de la réserpine.

3. Solutions stables selon la revendication 2, caractérisée en ce que, comme substances actives médicamenteuses, elles contiennent de la réserpine et des diurétiques.

4. Solutions stables selon la revendication 3, caractérisée en ce que, comme substances actives médicamenteuses, elles contiennent de la réserpine et du Clopamid [N-(cis-2,6-diméthylpipéridino)-4-chloro-3-sulfamoyl-benzamide].

5. Solutions stables selon la revendication 2, caractérisées en ce que, comme substances actives médicamenteuses, elles contiennent de la réserpine et d'autres antihypertoniques habituels.

6. Solutions stables selon la revendication 5, caractérisée en ce que, comme substances actives médicamenteuses, elles contiennent de la réserpine et des alcaloïdes de seigle ergoté.

7. Solutions stables selon la revendication 6, caractérisées en ce que, comme substances actives médicamenteuses, elles contiennent de la réserpine et un mélange de dihydroergocristine, de dihydroergocornine, de dihydroergocryptine sous forme de méthane-sulfonates, de préférence, dans le rapport de 1 : 1 : 1.

8. Solutions stables selon la revendication 2, caractérisées en ce que, comme substances actives médicamenteuses, elles contiennent de la réserpine, le méthane-sulfonate de dihydroergocristine et le Clopamid [N-(cis-2,6-diméthylpipéridino)-4-chloro-3-sulfamoyl-benzamide].

9. Solutions stables selon la revendication 1 à 8, caractérisées en ce que, comme unisseur et agent stabilisant, elles contiennent de l'acide méthane-sulfonique.

10. Procédé de préparation de solutions stables selon les revendications 1 à 9, caractérisé en ce qu'on dissout des alcaloïdes de Rauwolfia et leurs sels seuls ou conjointement avec d'autres substances actives médicamenteuses appropriées dans des solvants organiques et/ou dans l'eau et on ajoute 0,05 à 2 % en poids d'acide méthane- ou éthane-sulfonique, de préférence, d'acide méthane-sulfonique.